# EUROPEAN PATENT SPECIFICATION

(11) **EP 3 669 813 B1**
(45) Date of publication and mention of the grant of the patent: **21.07.2021**
(21) Application number: 18425103.1
(22) Date of filing: 20.12.2018
(51) Int. Cl.: A61B 90/70, A61L 2/02, A61L 2/04, A61L 2/18, B01D 61/14, C02F 1/44, A61L 2/26, A61B 50/13, A47L 15/42, A47L 15/16, A61L 2/00

(54) **TROLLEY WITH INTEGRATED MICROFILTRATION SYSTEM FOR WASHING AND DISINFECTING MEDICAL ITEMS**
WAGEN MIT INTEGRIERTEM MIKROFILTRATIONSSYSTEM ZUM WASCHEN UND DESINFIZIEREN VON MEDIZINISCHEN ARTIKELN
CHARIOT COMPORTANT UN SYSTÈME DE MICROFILTRATION INTÉGRÉ POUR LE LAVAGE ET LA DÉSINFECTION DES ARTICLES MÉDICAUX

(43) Date of publication of application: 24.06.2020
(73) Proprietor: Bonferraro S.p.A., 37060 Bonferraro (VR) (IT)
(72) Inventor: GOBBI, Ezio, 46037 Roncoferraro MN (IT); BENFATTI, Oscar, 37063 Isola della Scala VR (IT)
(74) Representative: Concone, Emanuele

(56) References cited:
- DE-A1-102010 049 684
- DE-A1-102014 100 635
- DE-B3-102004 029 970
- DE-B3-102004 060 289
- Manual: "Gebrauchsanweisung Model A 204", , 1 November 2015 (2015-11-01), pages MIELE Professional, 1-148, XP055596455, Gütersloh, Deutschland Retrieved from the Internet: URL:https://www.miele.co.uk/pmedia/ZGA/TX2 070/10319470-000-00_10319470-00.pdf [retrieved on 2019-06-13]
- Manual: "Gebrauchsanweisung Filterrohr A800", MIELE Professional, 30 June 2017 (2017-06-30), pages 1-136, XP055596444, Güthersloh, Deutschland Retrieved from the Internet: URL:https://www.miele.co.uk/pmedia/ZGA/TX2 070/10318060-000-00_10318060-00.pdf [retrieved on 2019-06-13]

## Description

The present invention concerns washing and disinfection apparatuses (thermodisinfectors) for medical items such as robotic surgical instruments, cannulas for laparoscopy and the like, and in particular a trolley used to load the items to be washed into a thermodisinfector and provided with an integrated microfiltration system. In the following, specific reference will be made to this application of the trolley in question, but it is clear that what is being said can also be applied in other sectors with similar needs for microfiltration with easy and rapid replacement or cleaning of the microfilters.

It is known that a trolley for the washing and disinfection of medical items of this type traditionally includes one or more horizontal manifolds provided with flexible washing tubes, each manifold being fed by a single central connector that extends vertically along the entire height of the trolley, possibly through a horizontal washing channel placed between the manifold and the connector along a direction perpendicular to both. The trolley also includes a support and locking system for said medical items, to ensure that they do not move during the wash cycle.

Since robotic surgical instruments include micro-mechanisms with steel handling cables only a few tenths of a millimeter in diameter, it is absolutely necessary to prevent solid impurities from being carried thereon during the washing, which could result in malfunction and damage. Therefore, the washing liquid must be microfiltered and this is done, in the two prior art trolleys known to the applicant, by means of a microfilter located at the entrance of each manifold.

In a first trolley from Belimed AG (Ballwil, CH), each manifold is mounted directly on the central connector without a washing channel between them, and the microfilter is inserted axially into an enlarged-diameter end portion of the manifold which is also used for installation on the central connector. Such a configuration of the trolley is very simple but impractical, since the operator has to uninstall the manifold to access the microfilter, and then has to re-install it in the correct position so that the washing tubes have the right inclination for the connection to the instruments to be washed.

In a second trolley from Miele & Cie. KG (Gütersloh, DE), marketed as model A 502, the micro filter is disc-shaped and inserted transversely to the manifold between the proximal end thereof and a support integral with the washing channel and located on the internal side thereof. More specifically, said support and said end of the manifold have truncated cone shapes that are opposed at their major bases, and the microfilter is blocked between them by a clamp that engages peripheral flanges formed on said major bases. The clamp is divided into two halves hinged on the support and joined by a screw carried by a first half that is screwed into a threaded seat formed on the second half.

Therefore, also in this case the operator must first uninstall and then re-install the manifold to access the microfilter, but the operation is simpler because the uninstalled manifold is supported by a structure that allows only the axial sliding thereof preventing any rotation, so as to ensure a correct position of the tubes. Such a configuration is more convenient than the Belimed trolley configuration but it is also much more complex and expensive, and the use of a disc microfilter inserted in a biconical chamber entails a significant pressure drop.

Two additional drawbacks common to both types of trolleys stem from the fact that access to the filter requires moving the manifold. A first drawback is the movement of the washing tubes together with the manifold, whereby if the trolley has already been loaded, the washing tubes may no longer be correctly inserted in the instruments to be washed, with the risk of inadequate washing or the need to check all connections again. In addition, since the means for installing the manifold on the trolley are located adjacent to the support structure of the instruments to be washed, the operation of opening and closing the seat of the microfilter involves the risk that the operator comes into contact with a dirty surgical instrument already loaded on the trolley.

In a third trolley also from Miele & Cie. KG (Gütersloh, DE), marketed as model A 204, the microfilter is a tubular filter, marketed as model A800, that extends along the whole manifold in which it is axially inserted through the distal end thereof opposite to the proximal end where the manifold is connected to a washing channel. This same arrangement is also disclosed in the patent application DE 102014100635A1 in the name of Miele & Cie. KG, showing in detail how the filter is provided with a seal at its proximal end.

In this case, access to the filter does not require moving the manifold, but since the distal end thereof is located adjacent to the support structure of the instruments to be washed, the operation of inserting/removing the microfilter still involves the risk that the operator comes into contact with a dirty surgical instrument already loaded on the trolley. Moreover, the microfilter is rather expensive and delicate since it must be as long as the manifold, and when it is inserted/removed its seal at its proximal end is scraped along the inner surface of the whole manifold, including all the outlets leading to the washing tubes. This can result in damage to the seal, thus jeopardizing the reliability of the microfilter as to keeping unfiltered washing liquid from reaching the surgical instruments to be washed.

DE 102004029970B3, DE 102004060289B3 and DE 102010049684A1, all by the same applicant Robert Simmoteit, disclose further examples of flushing bars in which a filter can also be inserted or connected upstream.

The object of the present invention is therefore to provide a trolley that overcomes the above-described drawbacks of prior art trolleys. This object is achieved by means of a trolley in which a cylindrical microfilter is inserted axially into an end portion of one between the manifold or the washing channel, in a position adjacent to the zone of connection between said two elements, and is accessible through the end of the element receiving the microfilter that extends outward beyond said zone of connection, said end being closed by a hermetic cap mounted in a removable way. Other advantageous features are recited in the dependent claims.

The main advantage of the trolley according to this invention is to combine a simple and inexpensive structure with a high ease of use, thanks to the fact that access to the microfilter does not require to uninstall and displace the manifold. As a result, it is possible to dispense with the anti-rotation support structure of the Miele A 502 trolley, with respect to which it is also much easier to access the microfilter that moreover does not require any particular shape of the manifold or washing channel to be housed, thus reducing the pressure drops.

A second advantage of the present trolley resulting from the absence of displacement of the manifold is the elimination of the risks highlighted above in case of access to the microfilter when the trolley is already loaded, since the washing tubes are not moved.

A further advantage of this trolley is that the operator does not have to come close to the surgical instruments that may already be present on the trolley, thanks to the arrangement of the access point to the microfilter on the external perimeter of the trolley.

Still other advantages of the present trolley with respect to the Miele A 204 trolley are a shorter filter that is cheaper and tougher, optimised use of the space on the trolley, and a greater reliability of the sealing function since the seal does not scrape along the whole manifold but remains close to the insertion end.

These and other advantages and characteristics of the trolley according to the present invention will be evident to those skilled in the art from the following detailed description of some embodiments thereof with reference to the attached drawings in which:
Fig.1 is a rear perspective view of a trolley according to a first embodiment of the invention, with some elements removed and see-through portions for greater clarity;
Fig.2 is a magnified view of detail A of Fig.1;
Fig.3 is a view similar to the previous one with the elements of the microfiltration system in an exploded view;
Fig.4 is a view similar to Fig.2 of a second embodiment of the invention;
Fig.5 is a view similar to the previous one with the elements of the microfiltration system in an exploded view;
Fig.6 is a view similar to Fig.4 of a third embodiment of the invention;
Fig.7 is a view similar to the previous one with the elements of the microfiltration system in an exploded view; and
Fig.8 is a front perspective view of the trolley of Fig.1 provided with support accessories for 12 robotic surgical instruments shown loaded on the trolley.

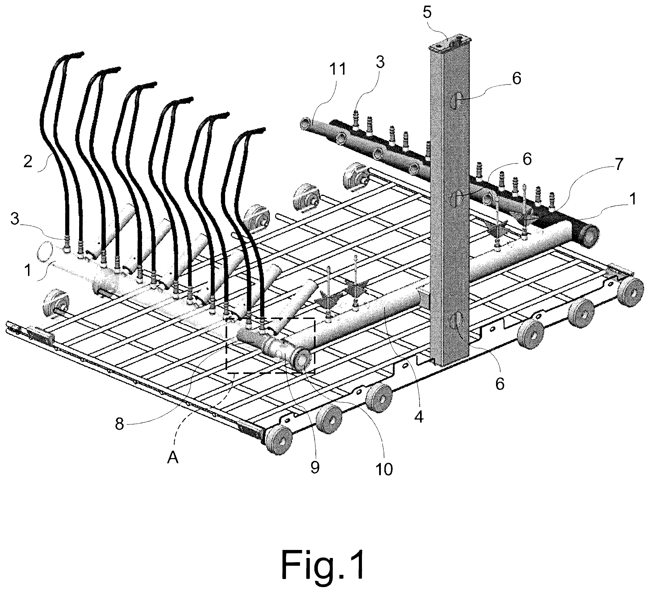

Referring to Fig.1, there is seen that a trolley for the washing and disinfection of medical items traditionally includes at least one horizontal cylindrical manifold 1 provided with flexible washing tubes 2 mounted on respective couplings 3 that extend radially in a substantially vertical position from a series of outlets arranged along a directrix of manifold 1. A horizontal washing channel 4 feeds manifold 1 being connected perpendicularly thereto in correspondence of an end portion of manifold 1, said washing channel 4 being in turn fed by a central connector 5 that extends vertically along the whole height of the trolley.

Note that the washing channel 4 is connected to the central connector 5 near an inlet 6 through which the washing/rinse fluid coming from the wash pump passes, and the central connector 5 can be provided with a plurality of inlets 6 (three in the example shown) for the connection to as many washing channels 4 (not shown). In addition, the washing channel 4 can be provided with a plurality of vertical nozzles 7 (four in the example shown) for washing items that do not require microfiltration such as, for example, trocar cannulas.

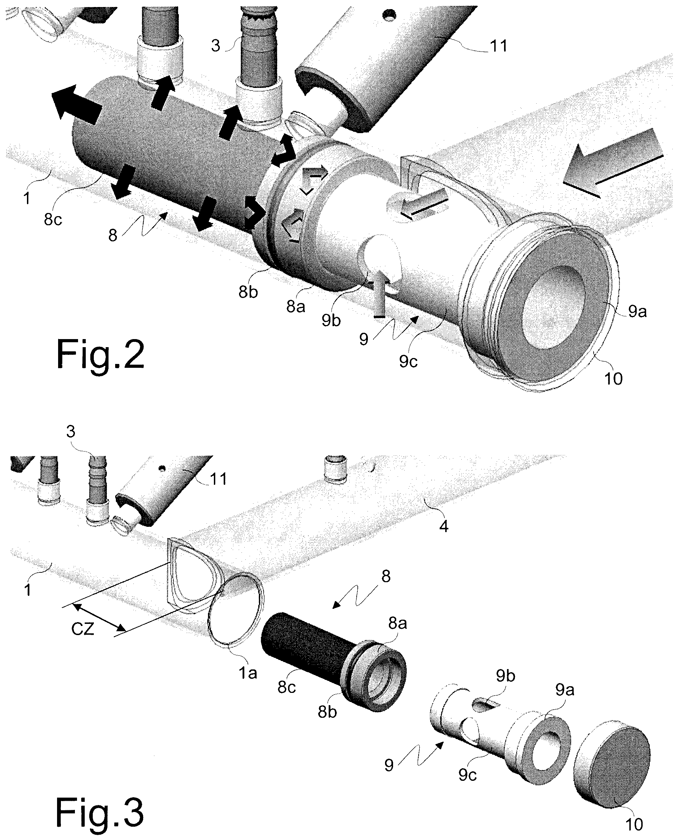

Referring now also to figures 2 and 3, there is seen that the main novel aspect of the trolley according to the invention lies in the microfiltration system which includes a cylindrical microfilter 8 mounted in a removable way on a hollow support 9. In this way, microfilter 8 can be inserted axially into an end portion of manifold 1 downstream (with respect to the flow direction of the fluid) of the connection zone CZ with the washing channel 4. It is possible to insert microfilter 8 through end 1a of manifold 1 because the latter extends outwards beyond said connection zone CZ and said end 1a is closed by a removable hermetic cap 10 (e.g. a screw cap).

More specifically, in the first embodiment shown in figures 1-3, the proximal end of the hollow support 9 has a flange 9a, which fits into a corresponding seat formed in end 1a of manifold 1, as well as a plurality of side openings 9b formed in its cylindrical body 9c extending through the connection zone CZ. Openings 9b allow the fluid coming from the washing channel 4 to reach microfilter 8 that is mounted, e.g. by means of a threaded coupling, on the distal end of the hollow support 9 (which could also have a simple grid structure instead of body 9c).

Microfilter 8 is provided at its proximal end with a sleeve 8a for reversible coupling with the hollow support 9, and a cylindrical microfiltering body 8c extends from sleeve 8a so that the fluid coming from body 9c of the hollow support 9 is microfiltered passing from the inside to the outside of microfilter 8. The sleeve 8a has a diameter such as to achieve a seal with manifold 1 thanks to the presence of an O-ring 8b, which is housed in a corresponding seat formed on the outer surface of said sleeve 8a.

In this way, as shown in Fig.2, a separation is obtained between the unfiltered water (grey arrows) and the microfiltered water (black arrows), the length of the hollow support 9 being such that the seal achieved by O-ring 8 is located in a position between the connection zone CZ and the first coupling 3.

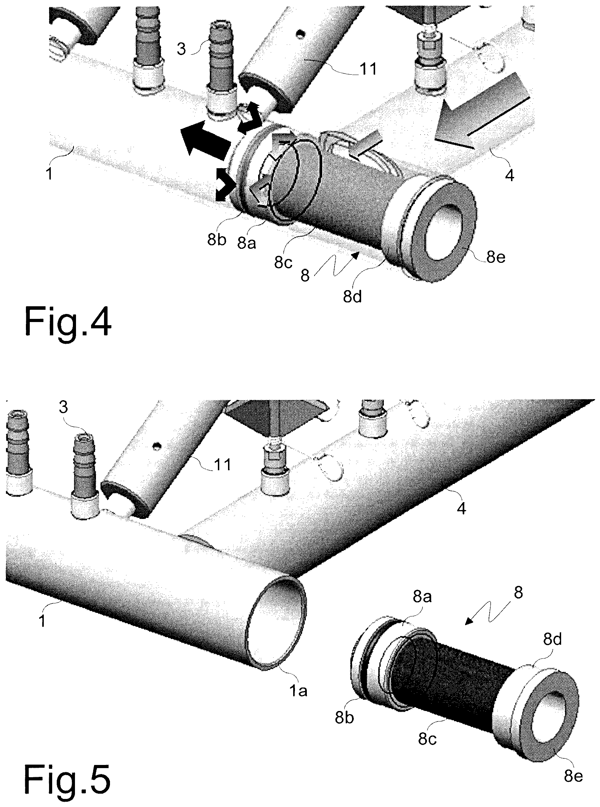

In the second embodiment illustrated in figures 4 and 5, microfilter 8 is inserted without the aid of the hollow support 9 because it is provided with a second sleeve 8d at the end opposite to that of sleeve 8a, said second sleeve 8d being provided with an end flange 8e for housing in the seat formed in end 1a (similarly to flange 9a described above). Therefore, in this case, the cylindrical microfilter body 8c extends between the two sleeves 8a, 8d and through the connection zone CZ, and the fluid coming from the washing channel 4 is microfiltered passing from the outside to the inside of the microfilter 8.

Moreover, filtration is facilitated by the higher pressure of the fluid that enters directly into microfilter 8 without the pressure losses due to the passage through the hollow support 9 and to the change of direction in the connection zone CZ. On the other hand, due to the presence of the second sleeve 8d, this version of microfilter 8 is more expensive than the version present in the first embodiment, and since microfilter 8 is replaced periodically it might be cheaper to use the less expensive microfilter mounted on the hollow support 9 (which is not replaced).

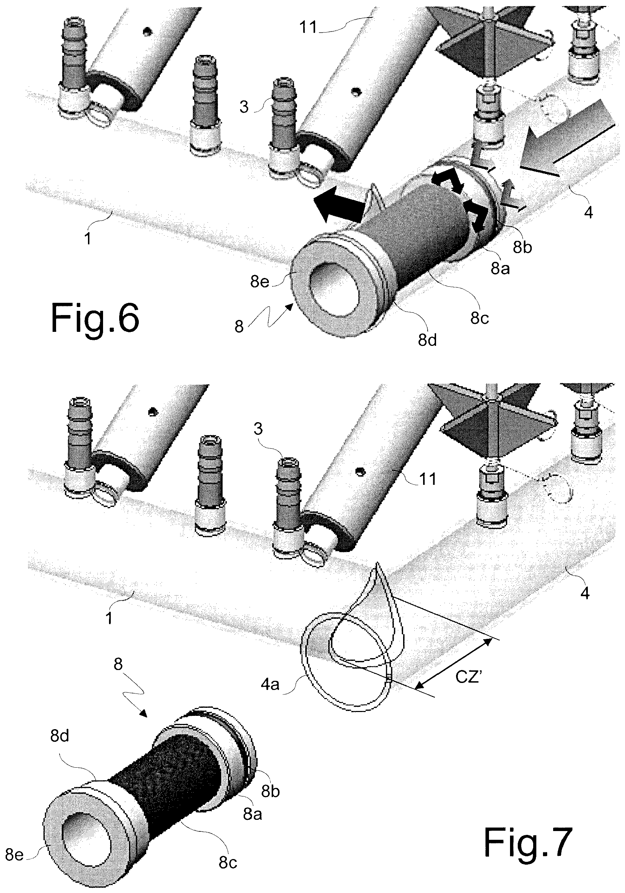

The third embodiment shown in figures 6 and 7 has a configuration similar to the second embodiment, with the difference of the inverted connection between the washing channel 4 and manifold 1, i.e. instead of being the former that connects to the latter (as in the first two embodiments) it is the latter that connects to the former. Consequently, microfilter 8 is inserted through end 4a of the washing channel 4 that extends beyond the connection zone CZ', with O-ring 8b that must be positioned in the washing channel 4 upstream of said connection zone CZ' with respect to the flow direction of the fluid coming from the central connector 5.

In this case, the fluid coming from the washing channel 4 is microfiltered passing from the inside to the outside of microfilter 8, and the seat for the end flange 8e is formed in end 4a of the washing channel 4. Moreover, filtration is made even easier by the higher pressure of the fluid that enters axially into the cylindrical microfiltering body 8c, instead of transversely as in the second embodiment.

Note that a fourth embodiment, not illustrated, could combine the connection between the washing channel 4 and manifold 1 of the third embodiment with the assembly microfilter 8+hollow support 9 of the first embodiment. Furthermore, in the first and fourth embodiments a microfilter 8 with double sleeves 8a, 8d could be used as in the second and third embodiments, but in this case sleeve 8d would obviously have no flange 8e.

From the description above it is clear that the wash/rinse fluid is entirely microfiltered through microfilter 8 before reaching couplings 3, then passing through the washing tubes 2 that lead it inside the instruments to be washed. Note that couplings 3 are typically made in pairs (six pairs in the example shown) because each robotic surgical instrument has a primary irrigation port and a secondary irrigation port, a washing tube 2 being inserted into each of these irrigation ports.

Thanks to this innovative configuration of the microfiltration system, microfilter 8 is extremely easy to uninstall and inspect as it is sufficient to unscrew cap 10 and extract the hollow support 9 on which microfilter 8 is mounted, or directly microfilter 8 in the absence of the hollow support 9, which can then be cleaned or replaced at each wash cycle even if the trolley is already loaded with instruments. Furthermore, the trolley preferably includes an identical second manifold 1, with its own microfilter 8, symmetrically arranged at the opposite end of the washing channel 4, so as to double the number of instruments that can be washed at each wash cycle.

Another innovative aspect of the present trolley, in the above-mentioned preferred configuration with two opposite manifolds 1 for each washing channel 4, lies in the fact that each manifold 1 is preferably also provided with a second series of outlets arranged along a directrix of manifold 1 rotated inwards by about 45°±10° compared to the first series of outlets on which couplings 3 are mounted. More specifically, this second series of outlets carries radial cylindrical sleeves 11 provided with internal nozzles, the number of outlets being equal to half of couplings 3 and each sleeve 11 being located between the two couplings 3 of a pair as described above.

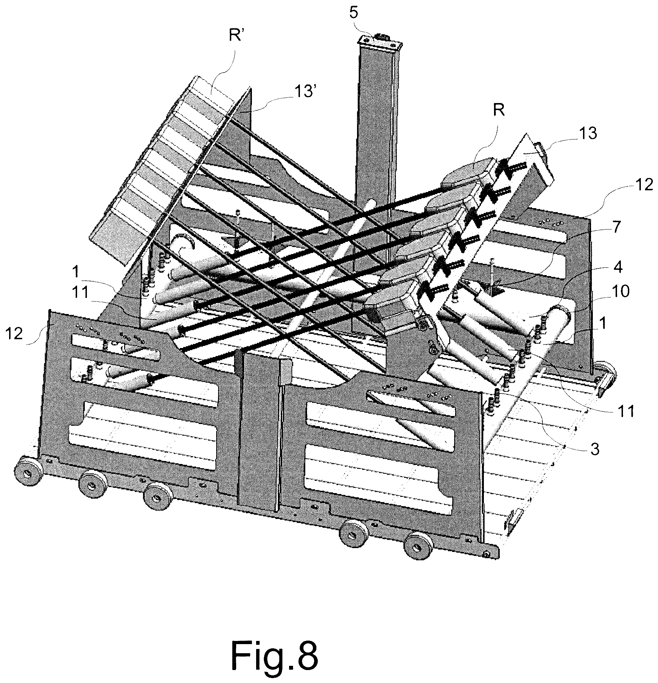

In this way, as shown in Fig.8, it is also possible to wash externally the ends of the instruments by inserting them in sleeves 11 that extend from the manifold 1 opposite to the one to which the washing tubes 2 (not shown in Fig.8) are connected that supply the fluid for the internal washing through the irrigation ports. In practice, the trolley is also provided with terminal walls 12, on the side of the central connector 5 and on the opposite side, which carry special supports on which to mount the instruments to be washed, which can also be of a different type. For example, in the configuration illustrated in Fig.8, a first type of instruments R are mounted on a first type of support 13 and a second type of instruments R' are mounted on a second type of support 13', the rods of instruments R, R' crossing so as to fit into sleeves 11 on the opposite side of the trolley.

It is clear that the embodiments of the trolley according to the invention described and illustrated above are only examples susceptible to numerous variations. In particular, the exact shape and arrangement of the components may vary somewhat depending on specific production requirements, as long as the general structure illustrated above is maintained. For example, although components 1-4 and 8-11 are shown to have a circular section, there is nothing to prevent them from being made with sections of other shapes, and also the horizontal/vertical arrangement of elements 1-5 can be changed, as can the orthogonality of the connections of the washing channel 4 with manifolds 1 and central connector 5.

Moreover, the reversible coupling between the hollow support 9 and microfilter 8 can be realized in other ways (snap-on, bayonet, etc.), and the seal can be achieved between the hollow support 9 and manifold 1 instead of between microfilter 8 and manifold 1. In another alternative version, elements 8 and 9 could be combined into a single element having a microfiltering part at least downstream of the seal region but possibly also upstream of it. Furthermore, the position of the sealing region can be defined in other ways that are technically equivalent to flanges 8e, 9a shown above, e.g. by having sleeve 8a abut against an end stop protruding inside manifold 1 or the washing channel 4.

Finally, if only the instruments connected to one of manifolds 1 are to be processed, it is possible to introduce in the other opposite manifold 1 a blind insert instead of microfilter 8 in order to block the access of the fluid. In this case, obviously, sleeves 11 for the external washing of the rod ends would not work on the plugged manifold 1, but if such washing were required then micro filter 8 could be simply omitted since no microfiltered water is required for this type of washing.

## Claims

1. Trolley for a washing and disinfecting apparatus, said trolley comprising at least one manifold (1) provided with at least a first series of radial outlets arranged along said at least one manifold (1), at least one washing channel (4) that feeds the at least one manifold (1), a central connector (5) that feeds said at least one washing channel (4) receiving wash/rinse fluid through at least one inlet (6), and at least one microfilter (8) that filters said wash/rinse fluid before it reaches said at least first series of radial outlets, the washing channel (4) being connected at a first end thereof to the manifold (1) at a connection zone (CZ) in an end portion of the manifold (1) or the manifold (1) being connected at a first end thereof to the washing channel (4) at a connection zone (CZ') in an end portion of the washing channel (4), wherein said microfilter (8) is inserted axially into said end portion of the manifold (1) or of the washing channel (4) and is accessible through the end (1a; 4a) of the manifold (1) or of the washing channel (4) extending outwards beyond said connection zone (CZ; CZ'), said end (1a; 4a) being closed by a removable hermetic cap (10), sealing means that provide a separation between the unfiltered wash/rinse fluid and the microfiltered one being located downstream of the connection zone (CZ) when the microfilter (8) is inserted in the manifold (1) or upstream of the connection zone (CZ') when the microfilter (8) is inserted in the washing channel (4), the position of said sealing means being defined by positioning means.

2. Trolley according to claim 1, **characterized in that** it further includes a hollow support (9) sized to be inserted axially into said end portion of the manifold (1) or of the washing channel (4) and to extend from the end (1a; 4a) thereof through the connection zone (CZ; CZ'), said hollow support (9) being provided at its distal end with means for a reversible coupling with the microfilter (8) that has corresponding means of reversible coupling, preferably of the threaded type.

3. Trolley according to claim 1 or 2, **characterized in that** the microfilter (8) consists of a cylindrical microfiltering body (8c) provided with one or two end sleeves (8a, 8d), the microfilter (8) being preferably sized to extend from the end (1a; 4a) of the manifold (1) or of the washing channel (4) through the connection zone (CZ; CZ').

4. Trolley according to any of the preceding claims, **characterized in that** the sealing means consist of an O-ring (8b) housed in a seat formed in the outer surface of a sleeve (8a), which is preferably arranged at one end of the microfilter (8).

5. Trolley according to any of the preceding claims, **characterized in that** the positioning means consist of a flange (8e; 9a) that fits into a seat formed in the end (1a; 4a) of the manifold (1) or of the washing channel (4), said flange (8e; 9a) being formed at one end of the microfilter (8) or of the hollow support (9), and/or consist of an end stop protruding inside the manifold (1) or the washing channel (4).

6. Trolley according to any of the preceding claims, **characterized in that** it further includes an identical second manifold (1), provided with its own microfilter (8), arranged symmetrically at the opposite end of the washing channel (4).

7. Trolley according to the previous claim, **characterized in that** each manifold (1) is provided with a second series of radial outlets arranged along the manifold (1) with an inward inclination of about 45°±10° with respect to the vertical, said second series of radial outlets carrying radial cylindrical sleeves (11) provided with internal nozzles.

8. Trolley according to the previous claim, **characterized in that** on each manifold (1) the outlets of the first series are arranged in pairs that extend radially in a substantially vertical position, each of said outlets being provided with a coupling (3) on which there is mounted a flexible washing tube (2), and **in that** the outlets of the second series are in number equal to half of the outlets of the first series and each sleeve (11) is located between the two couplings (3) of a pair.

9. Trolley according to any of the preceding claims, **characterized in that** it further includes additional washing channels (4) arranged parallel to each other along the central connector (5), each of said washing channels (4) feeding one or two manifolds (1).

10. Trolley according to the previous claim, **characterized in that** the central connector (5) is provided with a plurality of inlets (6) in number equal to the washing channels (4) and located respectively near each of them.

11. Apparatus for the washing and disinfection of medical items, **characterized in that** it includes a trolley according to any of the previous claims.

## Patentansprüche

1. Wagen für Wasch- und Desinfektionsvorrichtung, wobei der besagte Wagen wenigstens einen Verteiler (1) umfasst, der mit wenigstens einer ersten Reihe von radialen Auslässen versehen ist, die entlang des besagten wenigstens einen Verteilers (1) angeordnet sind, wenigstens einen Waschkanal (4), der den wenigstens einen Verteiler (1) speist, einen zentralen, den besagten wenigstens einen Waschkanal (4) speisenden Anschluss (5), der ein Wasch-/Spülfluid über wenigstens einen Einlass (6) empfängt, und wenigstens einen Mikrofilter (8), der das besagte Wasch-/Spülfluid filtriert, bevor es die besagte wenigstens eine erste Reihe von radialen Auslässen erreicht, wobei der Waschkanal (4) mit einem ersten Ende an dem Verteiler (1) in der Verbindungszone (CZ) im Endbereich des Verteilers (1) angeschlossen ist, oder wobei der Verteiler (1) mit einem ersten Ende an dem Waschkanal (4) in der Verbindungszone (CZ') in einem Endbereich des Waschkanals (4) angeschlossen ist, wobei der besagte Mikrofilter (8) in den besagten Endbereich des Verteilers (1) oder des Waschkanals (4) axial eingesteckt ist und durch das Ende (1a; 4a) des Verteilers (1) oder des Waschkanals (4), der sich nach außen bis jenseits der besagten Verbindungszone (CZ; CZ') erstreckt, erreichbar ist, wobei das besagte Ende (1a; 4a) durch eine lösbare, luftdichte Kappe (10) verschlossen ist, wobei ein Dichtungsmittel, das eine Trennung zwischen dem unfiltrierten Wasch-/Spülfluid und dem mikrofiltrierten Wasch-/Spülfluid bewirkt, stromabwärts der Verbindungszone (CZ) angeordnet ist, wenn der Mikrofilter (8) in den Verteiler (1) eingesteckt ist, oder stromaufwärts der Verbindungszone (CZ') angeordnet ist, wenn der Mikrofilter (8) in den Waschkanal (4) eingesteckt ist, wobei die Position des besagten Dichtungsmittels durch ein Positioniermittel vorgegeben ist.

2. Wagen gemäß Anspruch 1, **dadurch gekennzeichnet, dass** er zusätzlich einen hohlen Träger (9) aufweist, der entsprechend dimensioniert ist, um in den besagten Endbereich des Verteilers (1) oder des Waschkanals (4) axial eingesetzt zu werden und sich von dessen Ende (1a; 4a) durch die Verbindungszone (CZ; CZ') zu erstrecken, wobei der besagte hohle Träger (9) an seinem distalen Ende mit einem Mittel zur reversiblen Verbindung mit dem Mikrofilter (8) versehen ist, der ein entsprechendes Mittel zur reversiblen Verbindung, bevorzugt mit einem Gewinde, aufweist.

3. Wagen gemäß Anspruch 1 oder 2, **dadurch gekennzeichnet, dass** der Mikrofilter (8) aus einem zylindrischen mikrofiltrierenden Körper (8c) besteht, der mit einer oder zwei Endhülsen (8a, 8d) versehen ist, wobei der Mikrofilter (8) bevorzugt derart dimensioniert ist, um sich von dem Ende (1a; 4a) des Verteilers (1) oder des Waschkanals (4) durch die Verbindungszone (CZ; CZ') zu erstrecken.

4. Wagen gemäß einem der vorhergehenden Ansprüche, **dadurch gekennzeichnet, dass** das Dichtungsmittel aus einem O-Ring (8b) besteht, der in einem Sitz aufgenommen ist, die in der äußeren Oberfläche einer Hülse (8a) ausgebildet ist, die bevorzugt an einem Ende des Mikrofilters (8) angeordnet ist.

5. Wagen gemäß einem der vorhergehenden Ansprüche, **dadurch gekennzeichnet, dass** das Positioniermittel aus einem Flansch (8e; 9a), besteht, der in einen am Ende (1a; 4a) des Verteilers (1) oder des Waschkanals (4) ausgeformten Sitz hineinpasst, wobei der besagte Flansch (8e; 9a) an einem Ende des Mikrofilters (8) oder des hohlen Trägers (9) ausgeformt ist, und/oder aus einem Endanschlag besteht, der im Inneren des Verteilers (1) oder des Waschkanals (4) hervorsteht.

6. Wagen gemäß einem der vorhergehenden Ansprüche, **dadurch gekennzeichnet, dass** er ferner einen identischen zweiten Verteiler (1) aufweist, der mit seinem eigenen Mikrofilter (8) versehen ist und symmetrisch an dem entgegengesetzten Ende des Waschkanals (4) angeordnet ist.

7. Wagen gemäß dem vorhergehenden Anspruch, **dadurch gekennzeichnet, dass** jeder Verteiler (1) mit einer zweiten Reihe radialer Auslässe versehen ist, die entlang des Verteilers (1) mit einer nach inneren gerichteten Neigung von etwa 45°±10° gegenüber der Vertikalen angeordnet sind, wobei die besagte zweite Reihe von radialen Auslässen radiale, zylindrische Hülsen (11) trägt, die mit inneren Düsen ausgestattet sind.

8. Wagen gemäß dem vorhergehenden Anspruch, **dadurch gekennzeichnet, dass** an jedem Verteiler (1) die Auslässe der ersten Reihe in Paaren angeordnet sind, die sich radial in einer substantiell vertikalen Position erstrecken, wobei jeder der besagten Auslässe mit einer Kupplung (3) versehen ist, an der ein biegsames Waschrohr (2) montiert ist, sowie dadurch, dass die Auslässe der zweiten Reihe in der Anzahl gleich der Hälfte der Auslässe der ersten Reihe ist, und jede Hülse (11) sich zwischen zwei Kupplungen (3) eines Paars befindet.

9. Wagen gemäß einem der vorhergehenden Ansprüche, **dadurch gekennzeichnet, dass** er zusätzlich weitere Waschkanäle (4) umfasst, die entlang des zentralen Anschlusses (5) parallel zueinander angeordnet sind, wobei jeder der besagten Waschkanäle (4) einen oder zwei Verteiler (1) versorgt.

10. Wagen gemäß dem vorhergehenden Anspruch, **dadurch gekennzeichnet, dass** der zentrale Anschluss (5) mit einer Mehrzahl von Einlässen (6) versehen ist, deren Anzahl gleich der Anzahl der Waschkanäle (4) ist, und die sich jeweils in der Nähe von jedem derselben befinden.

11. Vorrichtung zum Waschen und Desinfizieren von medizinischen Artikeln, **dadurch gekennzeichnet, dass** sie einen Wagen gemäß einem der vorhergehenden Ansprüche enthält.

## Revendications

1. Chariot pour un appareil de lavage et de désinfection, ledit chariot comprenant au moins un collecteur (1) prévu avec au moins une première série de sorties radiales agencées le long dudit au moins un collecteur (1), au moins un canal de lavage (4) qui alimente le au moins un collecteur (1), un connecteur central (5) qui alimente ledit au moins un canal de lavage (4) recevant le fluide de lavage/rinçage à travers au moins une entrée (6), et au moins un microfiltre (8) qui filtre ledit fluide de lavage/rinçage avant qu'il n'atteigne ladite au moins une première série de sorties radiales, le canal de lavage (4) étant raccordé, au niveau de sa première extrémité, au collecteur (1), au niveau d'une zone de raccordement (CZ) dans une partie d'extrémité du collecteur (1) ou le collecteur (1) étant raccordé, au niveau de sa première extrémité, au canal de lavage (4) au niveau d'une zone de raccordement (CZ') dans une partie d'extrémité du canal de lavage (4), dans lequel :
ledit microfiltre (8) est inséré dans ladite partie d'extrémité du collecteur (1) ou du canal de lavage (4) et est accessible par l'extrémité (1a ; 4a) du collecteur (1) ou du canal de lavage (4) s'étendant vers l'extérieur au-delà de ladite zone de raccordement (CZ ; CZ'), ladite extrémité (1a ; 4a) étant fermée par un capuchon hermétique amovible (10), des moyens d'étanchéité qui fournissent une séparation entre le fluide de lavage/rinçage non filtré et le fluide microfiltré étant positionnés en aval de la zone de raccordement (CZ) lorsque le microfiltre (8) est inséré dans le collecteur (1) ou en amont de la zone de raccordement (CZ') lorsque le microfiltre (8) est inséré dans le canal de lavage (4), la position desdits moyens d'étanchéité étant définie par des moyens de positionnement.

2. Chariot selon la revendication 1, **caractérisé en ce qu'**il comprend un support creux (9) dimensionné pour être inséré axialement dans ladite partie d'extrémité du collecteur (1) ou du canal de lavage (4) et s'étendre à partir de son extrémité (1a; 4a) en passant par la zone de raccordement (CZ ; CZ'), ledit support creux (9) étant prévu au niveau de son extrémité distale avec des moyens pour un couplage réversible avec le microfiltre (8) qui a des moyens correspondants de couplage réversible, de préférence de type fileté.

3. Chariot selon la revendication 1 ou 2, **caractérisé en ce que** le microfiltre (8) se compose d'un corps de microfiltration cylindrique (8c) prévu avec un ou deux manchons d'extrémité (8a, 8d), le microfiltre (8) étant de préférence dimensionné pour s'étendre à partir de l'extrémité (1a ; 4a) du collecteur (1) ou du canal de lavage (4) en passant par la zone de raccordement (CZ ; CZ').

4. Chariot selon l'une quelconque des revendications précédentes, **caractérisé en ce que** les moyens d'étanchéité se composent d'un joint torique (8b) logé dans un siège formé dans la surface externe d'un manchon (8a), qui est de préférence agencé au niveau d'une extrémité du microfiltre (8).

5. Chariot selon l'une quelconque des revendications précédentes, **caractérisé en ce que** les moyens de positionnement se composent d'une bride (8e ; 9a) qui se monte dans un siège formé dans l'extrémité (1a ; 4a) du collecteur (1) ou du canal de lavage (4), ladite bride (8e ; 9a) étant formée au niveau d'une extrémité du microfiltre (8) ou du support creux (9) et/ou se composent d'une butée d'extrémité faisant saillie à l'intérieur du collecteur (1) ou du canal de lavage (4).

6. Chariot selon l'une quelconque des revendications précédentes, **caractérisé en ce qu'**il comprend en outre un second collecteur identique (1) prévu avec son propre microfiltre (8) agencé de manière symétrique au niveau de l'extrémité opposée du canal de lavage (4).

7. Chariot selon la revendication précédente, **caractérisé en ce que** chaque collecteur (1) est prévu avec une seconde série de sorties radiales agencées le long du collecteur (1) avec une inclinaison vers l'intérieur d'environ 45°±10° par rapport à la verticale, ladite seconde série de sorties radiales portant des manchons cylindriques radiaux (11) prévus avec des buses internes.

8. Chariot selon la revendication précédente, **caractérisé en ce que** sur chaque collecteur (1), les sorties de la première série sont agencées en paires qui s'étendent radialement dans une position sensiblement verticale, chacune desdites sorties étant prévues avec un couplage (3) sur lequel on monte un tube de lavage flexible (2) et **en ce que** les sorties de la seconde série ont un nombre égal à la moitié des sorties de la première série et chaque manchon (11) est positionné entre les deux couplages (3) d'une paire.

9. Chariot selon l'une quelconque des revendications précédentes, **caractérisé en ce qu'**il comprend en outre des canaux de lavage supplémentaires (4) agencés parallèlement entre eux le long du connecteur central (5), chacun desdits canaux de lavage (4) alimentant un ou deux collecteurs (1).

10. Chariot selon la revendication précédente, **caractérisé en ce que** le connecteur central (5) est prévu avec une pluralité d'entrées (6) en nombre égal aux canaux de lavage (4) et positionnées respectivement à proximité de chacun d'entre eux.

11. Appareil pour laver et désinfecter des articles médicaux, **caractérisé en ce qu'**il comprend un chariot selon l'une quelconque des revendications précédentes.
